Europäisches Patentamt

European Patent Office

Office européen des brevets

(11) Publication number: **0 330 712 B1**

## EUROPEAN PATENT SPECIFICATION

(45) Date of publication of patent specification: **26.08.92**  (51) Int. Cl.⁵: **A61M  25/00**, A61B 1/00

(21) Application number: **88103106.6**

(22) Date of filing: **02.03.88**

(54) **System for controlling shape and direction of a catheter,cannula,electrode,endoscope or similar article.**

(43) Date of publication of application:
**06.09.89 Bulletin  89/36**

(45) Publication of the grant of the patent:
**26.08.92 Bulletin  92/35**

(84) Designated Contracting States:
**AT CH DE IT LI SE**

(56) References cited:
**EP-A- 0 199 870**
**GB-A- 2 175 505**
**US-A- 3 773 034**

(73) Proprietor: **Bremer, Paul W.**
**433 Margaret Street**
**Jacksonville Florida 32204(US)**

(72) Inventor: **Bremer, Paul W.**
**433 Margaret Street**
**Jacksonville Florida 32204(US)**

(74) Representative: **UEXKÜLL & STOLBERG Paten-**
**tanwälte**
**Beselerstrasse 4**
**W-2000 Hamburg 52(DE)**

## Description

### BACKGROUND

This invention relates to a catheter assembly according to the first part of claim 1. Such a catheter assembly is disclosed in U.S. Patent No. 4,559,951.

Of great concern with such devices is their flexibility and steerability, characteristics which significantly bear on the ease with which the device can be introduced and passed through the various channels of the human, or animal body.

Various attempts have been made to produce a catheter, cannulae, endoscope or the like which is readily insertable and manipulatable for ease of advancement through body cavities or channels. See U.S. Patent No. 4,543,090 which corresponds to EP-A-0 199 870 and discloses a catheter having a distal end for ready insertion into a body, with a plurality of temperature-activated memory elements in its distal end. Each memory element assumes a first shape in response to temperature and a second shape in response to a force. The memory elements are coupled together and to a control means for deflecting the distal end of the catheter in a plurality of directions to steer or aim it within the body.

In U.S. Patent No. 4,176,662 an endoscope is disclosed which includes a propulsion system consisting of two radially expandable bladders separated by an axially expandable bellows. In this device, only the forward bladder is attached to the distal end of the endoscope so that by expanding and contracting the bladders in proper sequence, propulsion of the endoscope is achieved.

U.S. Patent No. 3,890,977 discloses a biological catheter or cannulae which incorporates material such as a titanium-nickel alloy having a heat activated shape memory. The device is formed and annealed at high temperature into a shape for effective anchoring or proper location in an organ or other structure of the body. At a temperature below its transitional temperature, it is reformed into a shape for ease of insertion, and when located as desired, it is heated above its transitional temperature to assume its proper anchoring or locating shape.

The catheter disclosed in U.S. Patent No. 3,773,034 bends the distal end of a catheter as desired, utilizing a fluid.

The catheter disclosed in U.S. Patent No. 3,674,014 includes permanent magnets and a magnetic field to bend the distal end of a catheter.

U.S. Patent No. 3,605,725 utilizes mechanically manipulatable control reins to steer a catheter, while U.S. Patent No. 3,043,309 discloses electromagnetic means for directing a tube in an intestinal intubation procedure.

Other prior art patents relevant to the use of heat activated shape memory alloy metals in medical devices include U.S. Patent Nos. 4,556,050 and 4,411,655.

The principal object of this invention is to utilize shape memory metal technology to provide a steerable catheter, cannulae, electrode, endoscope and the like for easy insertion and advancement within the body.

To this end, the invention relates to a catheter assembly comprising an elongated, flexible tubular body including an inner flexible tube, said tubular body including a proximal end for connection to a control means including a power source, and a distal end for insertion into a body;

a plurality of temperature activated shape memory wires extending along said flexible tubular body for effecting a change in shape of the distal end of the catheter; a plurality of electrical conductors extending between said shape memory wires and the power source; said control means adapted to selectively heat said shape memory wires to a predetermined temperature, the assembly characterized by a plurality of relatively rigid rings encircling said flexible tubular body at axially spaced locations adjacent the distal end thereof, said plurality of shape memory wires extending between said plurality of rings and being individually contractible upon heating to said predetermined temperature to effect a change in shape of the distal end of the catheter. The elongated, flexible tubular body is preferably of multi-layer construction including an inner tubular member of relatively stiff plastic material which may enclose fiber optic bundles, air feed channels, electrodes and the like. A flat ribbon-type conductor is helically wrapped about the inner tubular member and covered with insulating material.

Fixedly attached to an outer surface of the insulating material at axially spaced locations in the distal end area of the tubular member are a plurality of rigid rings, preferably made of aluminum or plastic material. These rings may be fixed to the outer insulating surface by any suitable adhesives, such as known epoxy resins.

Individual titanium-nickel shape memory alloy wires extend between, and are attached to adjacent rings, each pair of rings defining a segment along the length of the catheter-like flexible probe. Each segment preferably has four titanium-nickel alloy wires, spaced 90° apart about the periphery of the probe and extending axially along the probe. The individual wires are electrically connected through the rigid ring to appropriate ones of the conductors beneath the insulating layer by laser welding or other suitable methods including mechanical crimping.

The conductors are connected at the proximal end of the tubular probe to a suitable control device, such as a microprocessor, which is operable to selectively heat various of the titanium-nickel alloy wires to a predetermined temperature which causes that wire to contract, i.e., shorten its length so as to bend the distal end of the probe in the desired direction.

By sequentially heating selected ones of said wires, it is possible to steer the probe to the desired location within the body.

Further objects and advantages of the invention will become apparent upon consideration of the drawings, detailed description and claims which follow.

## BRIEF DESCRIPTION OF THE DRAWINGS

FIGURE 1 is a perspective view of a medical probe device in accordance with an exemplary embodiment of the invention, with portions removed to show the internal construction thereof;
FIGURE 2 is a cross-sectional view of the probe device illustrated in FIGURE 1, and taken along the line II-II;
FIGURE 3 is a perspective view of the medical probe of this invention connected to a microprocessor control device shown in schematic form.

## DETAILED DESCRIPTION OF THE DRAWINGS

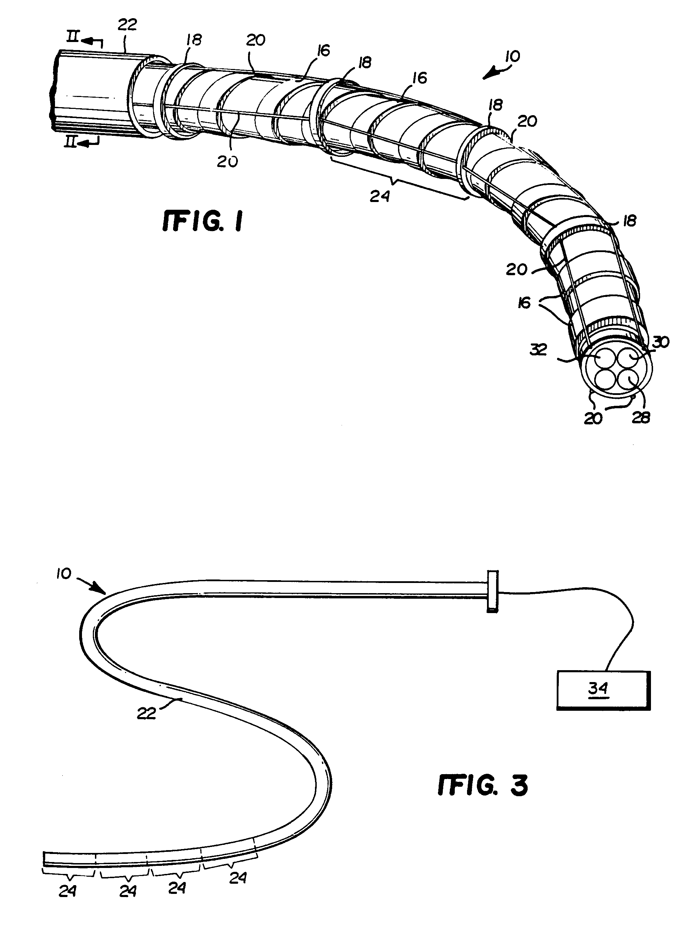

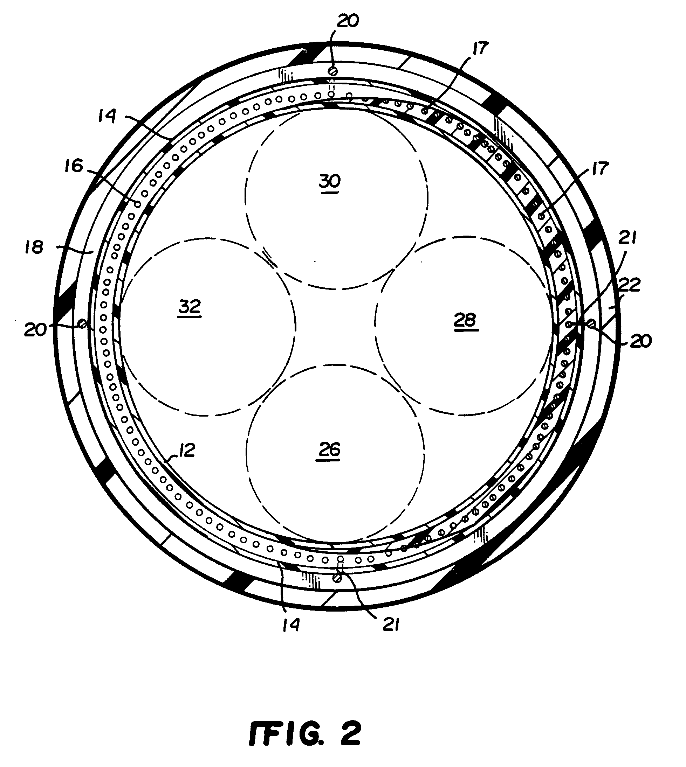

Referring now to FIGURES 1 and 2, there is shown the distal end of the catheter, cannulae, endoscope or other medical probe device generally referred to by the reference numeral 10. For convenience sake, the probe will hereinafter be referred simply as a catheter, it being understood that the invention applies equally as well to other medical probe devices including those specifically mentioned above as well as others of a similar nature which are designed for insertion within the human or animal body.

The catheter constructed in accordance with this invention is a multi-layered tubular member which includes an inner flexible tube 12 made of a suitable plastic material. Helically wound about the plastic inner tube 12 is a flat, ribbon-type conductor strip 16 of a conventional type which includes a plurality of electrical conductors 17 arranged in single or multi-layer form. The conductor strip is held in place with respect to the inner flexible tube 12 by a surrounding layer of electrical insulating material 14. It is to be understood that in FIGURE 1, the insulating material 14 has been removed to more clearly illustrate the relationship between the conductor strip 16, rigid rings 18 and wires 20, as further described hereinbelow.

Affixed to the outer surface of the insulating layer 14, and axially spaced along the distal end portion of the catheter, are a plurality of rigid, non-conductive rings 18. These rings, preferably made of plastic or aluminum, may be held in place by applying a suitable adhesive such as an epoxy resin to the inner surface of the rigid rings.

Extending between each rigid ring 18 is a set of wires, preferably made from a titanium-nickel shape memory alloy commercially marketed by the Toki Corporation under the name BIOMETAL® At least two wires, and preferably a minimum of four wires, extend between each adjacent pair of rigid rings. The four BIOMETAL® wires are preferably arranged 90° apart about the circumference of the rigid rings. At each end of the individual wires, an electrical connection 21 is made between the wire and an appropriate conductor within the flat, ribbon-type conductor strip by, for example, laser welding, or mechanical crimping. Additional wires may be added to each segment, depending on the degree of steering control desired.

Surrounding this structure is an outer relatively loosely fitting, flexible plastic sheath 22.

Within the multi-layer, tubular construction, there is shown in phantom in FIGURE 2, a plurality of tubular members 26, 28, 30 and 32 which may be a fiber optic bundle for illumination and/or viewing purposes, feed lines for air or other fluid, channels for the application of suction, or shape change metal wires used to control miniature surgical tools including cutting and grasping devices.

In the manufacture of the catheter device of this invention, various conventional processes may be utilized to arrive at the construction described hereinabove. For example, where a bundle such as that shown in phantom at 26, 28, 30 and 32 is to be enclosed within the probe, and because of the extremely small size of the probe, e.g., a diameter of from 6,25 to 9,4 mm (1/4 to 3/8 of an inch), the inner flexible tube portion 12 is preferably formed by dipping the bundle into a heated liquid vinyl composition to form a tubular body surrounding the bundle. After wrapping the flat, ribbon-type conductor strip 16 around the inner flexible tube 12, a similar dipping technique may be employed in order to form the insulating layer 14.

It is also to be understood that rather than a flat ribbon-type conductor, it may be advantageous to form the conductors between layers of insulating material utilizing conventional electroplating and etching techniques.

It is also important in the construction of the flexible, medical probe device of this invention that the inner flexible tube 12 be relatively stiff so as to be able to bend without collapsing, particularly when there is a fiber optic or other bundle within the tube. On the other hand, the outer flexible sheath 22 must be flexible enough to permit some collapse as the individual biometal wires contract to bend the distal end of the catheter.

Referring now to FIGURE 3, the catheter device is shown in operative connection to a microprocessor device 34 including a power source, which, as will be appreciated by those skilled in the art, may be programmed to selectively heat individual ones of the biometal wires within a particular segment 24 to cause the distal end of the catheter to bend in the desired direction.

In operation, once the catheter has been inserted in the particular body orifice, incision, artery, vein or other passage, microprocessor 34 is employed to apply very low pulse currents on the order of 3 ohms to selected ones of the biometal wires 20. Low amperage pulse currents are necessary to prevent hot spots from forming and melting the biometal wires. Upon heating to a predetermined temperature, the heated biometal wire will contract and thereby effect a slight bend in the distal end of the catheter. By sequentially heating selected ones of said biometal wires in the various segments, the catheter may be effectively steered in any direction throughout its path of travel in the body. Because selected wires in any or all of the segments may be heated, it is possible to steer the catheter through complex bends in the various body passages.

## Claims

1. A catheter assembly (10) comprising an elongated, flexible tubular body including an inner flexible tube (12), said tubular body including a proximal end for connection to a control means (34) including a power source, and a distal end for insertion into a body;

    a plurality of temperature activated shape memory wires (20) extending along said flexible tubular body for effecting a change in shape of the distal end of the catheter; a plurality of electrical conductors (17) extending between said shape memory wires (20) and the power source; said control means (34) adapted to selectively heat said shape memory wires (20) to a predetermined temperature, the assembly characterized by a plurality of relatively rigid rings (18) encircling said flexible tubular body at axially spaced locations adjacent the distal end thereof, said plurality of shape memory wires (20) extending between said plurality of rings (18) and being individually contractible upon heating to said predetermined temperature to effect a change in shape of the distal end of the catheter.

2. A catheter assembly as defined in claim 1 and further characterized by an outer flexible sheath (22) concentrically surrounding said flexible tubular body.

3. A catheter assembly as defined in claim 1 wherein said metal wires are composed of a titanium-nickel alloy.

4. A catheter assembly as defined in claim 1 wherein said plurality of sets of conductors (17) are electroplated between insulating layers of said multi-layered wall construction.

5. A catheter assembly as defined in claim 1 or 4 wherein said conductors (17) are provided in a flat ribbon-type conductor strip (16) helically wound within said flexible tubular body.

6. A catheter assembly as defined in claim 1 wherein said control means (34) includes a microprocessor.

7. A catheter assembly as defined in claim 1 wherein at least four temperature activated memory wires (20) extend between each pair of adjacent rigid rings (18).

8. A catheter assembly as defined in claim 1 wherein said flexible tubular body contains a tube bundle (26,18,30,32) therein.

9. A catheter assembly as defined in claim 10 wherein said tube bundle (26,28,30,32) comprises a fiber optic tube bundle.

10. A catheter assembly as recited in claim 1 wherein said elongated, flexible tubular member carries a fiber optic tube bundle (26,28,30,32).

11. A catheter assembly as recited in claim 1 wherein said control device (34) includes means for selectively applying a low pulse current to one or more of said memory wires (20) to effect a bend in the distal end of said probe device.

## Patentansprüche

1. Katheterbaugruppe (10) mit einem länglichen, flexiblen Rohrkörper mit einem inneren flexiblen Rohr (12), wobei dieser Rohrkörper ein proximales Ende für den Anschluß an Steuermittel (34) einschließlich einer Stromquelle und ein distales Ende für das Einführen in einen Körper einschließt; einer Vielzahl von temperaturaktivierten Formspeicherdrähten, (20), die sich entlang des flexiblen Rohrkörpers erstrecken, um eine Formänderung des distalen Katheterendes zu bewirken; einer Vielzahl von elektrischen Leitern (17), die sich zwischen den Formspeicherdrähten (20) und der Stromquelle erstrecken, wobei die die Steuermittel (34) die Formspeicherdrähte (20) auf eine vorbestimmte Temperatur erhitzen können, wobei die Baugruppe durch eine Vielzahl relativ starrer Ringe (18) **gekennzeichnet** ist, die den flexiblen Rohrkörper an axial beabstandeten Orten nahe dem distalen Ende umgeben und die Vielzahl von Formspeicherdrähten (20), die sich zwischen der Vielzahl der Ringe (18) erstrecken und nach Erhitzung auf die vorbestimmte Temperatur individuell zusammenziehbar sind, um eine Formänderung des distalen Katheters zu bewirken.

2. Katheterbaugruppe nach Anspruch 1, **dadurch gekennzeichnet,** daß eine äußere flexible Ummantelung (22) den flexiblen Rohrkörper konzentrisch umschließt.

3. Katheterbaugruppe nach Anspruch 1, **dadurch gekennzeichnet,** daß die Metalldrähte aus einer Titan-Nickel-Legierung bestehen.

4. Katheterbaugruppe nach Anspruch 1, **dadurch gekennzeichnet,** daß eine Vielzahl von Leitersätzen (17) zwischen den Isolierschichten der Mehrschicht-Wandkonstruktion elektroplattiert sind.

5. Katheterbaugruppe nach Anspruch 1, **dadurch gekennzeichnet,** daß die Leiter (17) in einem Leiter-Flachbandstreifen (16) wendelförmig im flexiblen Rohrkörper gewunden sind.

6. Katheterbaugruppe nach Anspruch 1, **dadurch gekennzeichnet,** daß die Steuerungsmittel (34) einen Mikroprozessor einschließen.

7. Katheterbaugruppe nach Anspruch 1, **dadurch gekennzeichnet,** daß sich mindestens vier temperaturaktivierte Speicherdrähte (20) zwischen jedem Paar von benachbarten starren Ringen erstrecken.

8. Katheterbaugruppe nach Anspruch 1, **dadurch gekennzeichnet,** daß der flexible Rohrkörper ein Rohrbündel (26,28,30,32) aufweist.

9. Katheterbaugruppe nach Anspruch 8, **dadurch gekennzeichnet,** daß das Rohrbündel (26,28,30,32) ein faseroptisches Bündel ist.

10. Katheterbaugruppe nach Anspruch 1, **dadurch gekennzeichnet,** daß das längliche, flexible Rohrbauteil ein faseroptisches Rohrbündel (26,28,30,32) trägt.

11. Katheterbaugruppe nach Anspruch 1, **dadurch gekennzeichnet,** daß das Steuergerät (34) Mittel für die selektive Aufbringung eines Niedrig-Impuls-Stromes auf einen oder mehrere Speicherdrähte (20) einschließt, um eine Biegung des distalen Sondenendes zu bewirken.

**Revendications**

1. Ensemble de cathéter (10) comprenant un corps tubulaire flexible allongé comportant un tube flexible intérieur (12), ledit corps tubulaire comportant une extrémité proximale pour la jonction à des moyens de commande (34) comportant une source de puissance électrique et une extrémité distale pour l'insertion dans un corps, une pluralité de fils mémoire de forme activée par la température (20) s'étendant le long du corps tubulaire flexible mentionné pour effectuer un changement de forme de l'extrémité distale du cathéter, une pluralité de conducteurs électriques (17) s'étendant entre les fils mémoire de forme mentionnés (20) et la source de puissance électrique, les moyens de commande mentionnés (34) étant adaptés pour chauffer de manière sélective les fils mémoire de forme mentionnés (20) à une température prédéterminée, l'ensemble étant **caractérisé par** une pluralité d'anneaux relativement rigides (18) encerclant ledit corps tubulaire flexible à des endroits espacés axialement adjacents à l'extrémité distale de celui-ci, ladite pluralité de fils mémoire de forme (20) s'étendant entre ladite pluralité d'anneaux (18) et étant individuellement susceptibles de contraction après chauffage à ladite température prédéterminée pour effectuer un changement de forme de l'extrémité distale du cathéter.

2. Ensemble de cathéter comme défini dans la revendication 1 et de plus caractérisé par une gaine flexible extérieure (22) entourant de manière concentrique ledit corps tubulaire flexible.

3. Ensemble de cathéter comme défini dans la revendication 1 dans lequel les fils de métal mentionnés sont composés d'un alliage titannickel.

4. Ensemble de cathéter comme défini dans la revendication 1 dans lequel ladite pluralité d'ensembles de conducteurs (17) est métallisée par galvanisation entre les couches isolantes de ladite construction de paroi à plusieurs couches.

5. Ensemble de cathéter comme défini dans la revendication 1 ou 4 dans lequel les conducteurs mentionnés (17) se trouvent dans une bande conductrice plate du type ruban (16) enroulée en spirale à l'intérieur du corps tubulaire flexible mentionné.

6. Ensemble de cathéter comme défini dans la revendication 1 dans lequel les moyens de commande mentionnés (34) comprennent un microprocesseur.

7. Ensemble de cathéter comme défini dans la revendication 1 dans lequel au moins quatre fils mémoire activés par la température (20) s'étendent entre chaque paire d'anneaux rigides adjacents (18).

8. Ensemble de cathéter comme défini dans la revendication 1 dans lequel ledit corps tubulaire flexible contient un faisceau tubulaire à l'intérieur (26, 28, 30, 32).

9. Ensemble de cathéter comme défini dans la revendication 10 dans lequel ledit faisceau tubulaire (26, 28, 30, 32) comprend un faisceau tubulaire de fibres optiques.

10. Ensemble de cathéter comme défini dans la revendication 1 dans lequel ledit organe tubulaire flexible allongé porte un faisceau tubulaire de fibres optiques (26, 28, 30, 32).

11. Ensemble de cathéter comme défini dans la revendication 1, dans lequel le dispositif de commande (34) comporte des moyens pour appliquer sélectivement un courant faiblement pulsé à l'un des fils mémoire mentionnés (20) ou à plusieurs pour effectuer une courbure dans l'extrémité distale du dispositif de sonde mentionné.

FIG. 1

FIG. 3

FIG. 2